# EUROPEAN PATENT SPECIFICATION

(11) **EP 2 089 077 B1**
(45) Date of publication and mention of the grant of the patent: **28.12.2016**
(21) Application number: 07838556.4
(22) Date of filing: 20.09.2007
(51) Int. Cl.: A61L 31/14, A61L 31/16, A61L 31/08

(54) **MEDICAL DEVICES HAVING BIODEGRADABLE POLYMERIC REGIONS WITH OVERLYING HARD, THIN LAYERS**
MEDIZINISCHE VORRICHTUNGEN MIT BIOLOGISCH ABBAUBAREN BEREICHEN MIT ÜBERLAPPENDEN HARTEN UND DÜNNEN SCHICHTEN
DISPOSITIFS MÉDICAUX COMPORTANT DES ZONES POLYMÈRES BIODÉGRADABLES RECOUVERTES DE COUCHES DURES ET FINES

(30) Priority: 20.09.2006 US 845954 P; 10.08.2007 US 891588
(43) Date of publication of application: 19.08.2009
(60) Divisional application: 10159669.0
(73) Proprietor: Boston Scientific Limited, Hamilton HM11 (BM)
(72) Inventor: WEBER, Jan, NL-6228 GJ Maastricht (NL)
(74) Representative: Peterreins Schley
(86) International application number: PCT/US2007/020368
(87) International publication number: WO 2008/036357

(56) References cited:
- EP-A- 0 824 900
- EP-A- 1 754 684
- EP-A- 1 980 223
- WO-A-01/80920
- WO-A-2006/061598
- WO-A-2008/034048
- US-A1- 2003 104 028
- US-A1- 2005 197 687
- US-A1- 2006 079 863

## Description

### FIELD OF THE INVENTION

The present invention relates to implantable or insertable medical devices according to claim 1.

### BACKGROUND OF THE INVENTION

Numerous polymer-based medical devices have been developed for implantation or insertion into the body. For example, in recent years, drug eluting coronary stents, which are commercially available from Boston Scientific Corp. (TAXUS), Johnson & Johnson (CYPHER) and others have become the standard of care for maintaining vessel patency. These existing products are based on metallic balloon-expandable stents with biostable polymer coatings, which release antiproliferative drugs at a controlled rate and total dose.

Biodegradable polymers, on the other hand, offer the prospect of reducing or eliminating long term effects that may be associated with biostable medical devices, because they are degraded over time.

US2003/104028 discloses coatings for medical devices comprising organic polymers, inter alia polylactide-co-glycolide and a diamon-like carbon layer.

### SUMMARY OF THE INVENTION

According to an aspect of the invention, medical devices as defined in claim 1 are provided.

An advantage of the present invention is that the hard, thin layer provides advantages attendant such a material (e.g., promotion of cell/tissue growth, etc.), whereas the biodegradable polymeric region provides advantages attendant a material that degrades over time (e.g., increased flexibility, etc.).

These and many other aspects, embodiments and advantages of the present invention will become readily apparent to those of ordinary skill in the art upon review of the Detailed Description and Claims to follow.

### BRIEF DESCRIPTION OF THE DRAWINGS

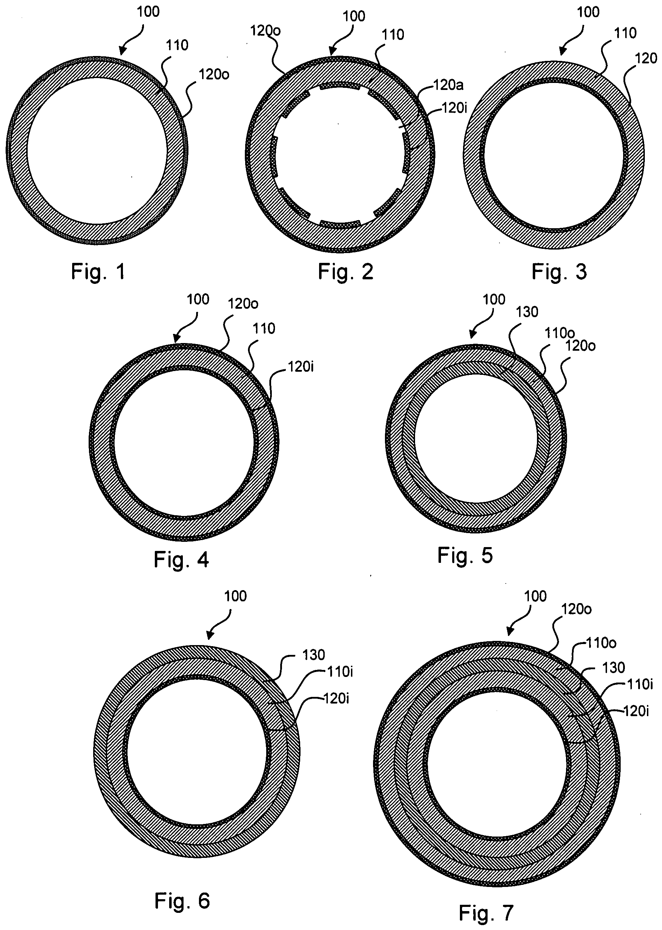

Only figure 2 is part of the invention.
Figs. 1-7 are schematic cross-sectional illustrations of tubular medical devices.
Fig. 8A is a schematic perspective view of stent.
Fig. 8B is a schematic cross-sectional view of the stent of Fig. 8A, taken along line a--a.
Fig. 8C is a schematic expanded top view of the rectangular region defined by dashed lines in Fig. 8A.
Fig. 9A is a schematic top view of a planar sheet, which may be rolled into a tubular medical device (i.e., a stent).
Fig. 9B is a schematic cross-sectional view of the sheet of Fig. 9A, taken along line a--a.
Fig. 10 is a schematic cross-sectional illustration of a tubular medical device.

### DETAILED DESCRIPTION OF THE INVENTION

According to one aspect of the invention, medical devices are provided as defined in claim 1.

Examples of medical devices to which the present invention is applicable include various implantable or insertable medical devices, for example, stents (including coronary vascular stents, peripheral vascular stents, cerebral, urethral, ureteral, biliary, tracheal, gastrointestinal and esophageal stents), stent grafts, vascular grafts, vascular access ports, catheters (e.g., renal or vascular catheters such as balloon catheters and various central venous catheters), and guide wires.

As used herein, a "thin" layer is one that is less than 5 µm in thickness, preferably from 1 µm to 500 nm to 250 nm to 100 nm to 25 nm to 10 nm or less, with the optimal thickness depending upon the specific medical device. Thinness is advantageous, for example, where it is desired that the hard, thin, biostable layers do not have a significant effect upon the initial bulk mechanical properties of the device, where it is desired that the medical device have a minimal effect upon adjacent tissue after the degradation of the biodegradable polymeric region and so forth. However, the layer should not be so thin that it is unable to withstand the rigors of device However, the layer should not be so thin that it is unable to withstand the rigors of device implantation/insertion or *in vivo* stresses such as those associated with polymer swelling and/or degradation.

A "hard" layer is one that has a surface Young's modulus of at least 50 MPa, preferably ranging from 50 MPa to 100 MPa to 300 MPa to 1 GPa to 3 GPa to 10 GPa to 30 GPa to 100 GPa or more.

As defined herein, a "biostable" region is one which remains intact over the time period that the medical device is intended to remain implanted within the body, typically over a period of at least 1 year. Similarly, as defined herein, a "biodegradable" region is one which does not remain intact over the period that the medical device is intended to remain within the body, for example, due to any of a variety of mechanisms including chemical breakdown, dissolution, and so forth. Depending upon the device within which the biodegradable region is disposed and the mechanism of degradation of the biodegradable region, the time period required to degrade at least 50 wt% of the biodegradable polymer within the device may vary, for example, from 1 day or less to 2 days to 4 days to 1 week to 2 weeks to 5 weeks to 10 weeks to 25 weeks to 1 year or longer.

Biodegradable polymeric regions in accordance with the present invention (along with their associated hard, thin layers) correspond to the entire device, e.g. a stent.

Some structures will now be described with reference to Figs. 1-7 which schematically illustrate cross-sections of tubular medical devices (or tubular portions thereof). Only Fig. 2 forms part of the present invention. Fig. 1 illustrates a medical device 100 that comprises a biodegradable polymeric region 110 having an outer hard, thin surface layer 120o. Fig. 3 illustrates a medical device 100 that comprises a biodegradable polymeric region 110 having an inner hard, thin surface layer 120i. Fig. 4 illustrates a medical device 100 that comprises a biodegradable polymeric region 110 having an inner hard, thin surface layer 120i and an outer hard, thin surface layer 120o.

Fig. 5 illustrates a medical device 100 that comprises a substrate 130, a biodegradable polymeric region 110o on an outer surface of the substrate 130, and a hard, thin surface layer 120o on an outer surface of the biodegradable polymeric region 110o. In this regard, medical device coatings are typically on the order of several microns in thickness, whereas hard, thin layers in accordance with the invention are typically less than a micron in thickness. Fig. 6 illustrates a medical device 100 that comprises a substrate 130, a biodegradable polymeric region 110i on an inner surface of the substrate 130, and a hard, thin surface layer 120i on an inner surface of the biodegradable polymeric region 110i. Fig. 7 illustrates a medical device 100 that comprises a substrate 130, a biodegradable polymeric region 110o on an outer surface of the substrate 130, a hard, thin surface layer 120o on an outer surface of the biodegradable polymeric region 110o, a biodegradable polymeric region 110i on an inner surface of the substrate 130, and a hard, thin surface layer 120i on an inner surface of the biodegradable polymeric region 110i.

In the present invention, the biostable hard, thin surface layer(s) cover(s) the entire surface of the biodegradable polymeric region. In the present invention, steps are taken to ensure that apertures are present in the inner hard, thin surface layer. For example, Fig. 2, like Fig. 4, illustrates a cross-section of a tubular medical device 100 that comprises a biodegradable polymeric region 110 with an inner hard, thin surface layer 120i and an outer hard, thin surface layer 120o. Unlike Fig. 4, which is not part of the invention, multiple apertures 120a are formed in the inner hard, thin surface layer 120i to promote biodegradation of the polymeric region 110.

Aperture shapes and sizes can vary widely and include, among many other possibilities, apertures in which the length and width are of similar scale and whose perimeter may be of irregular or regular geometry (e.g., circular, oval, triangular, square, rectangular, pentagonal, etc., apertures), and apertures in which the length significantly exceeds the width (e.g., in the form of stripes, etc.), which may be, for example, of constant or variable width, and may extend along the surface in a linear fashion or in a nonlinear fashion (e.g., serpentine, zigzag, etc.).

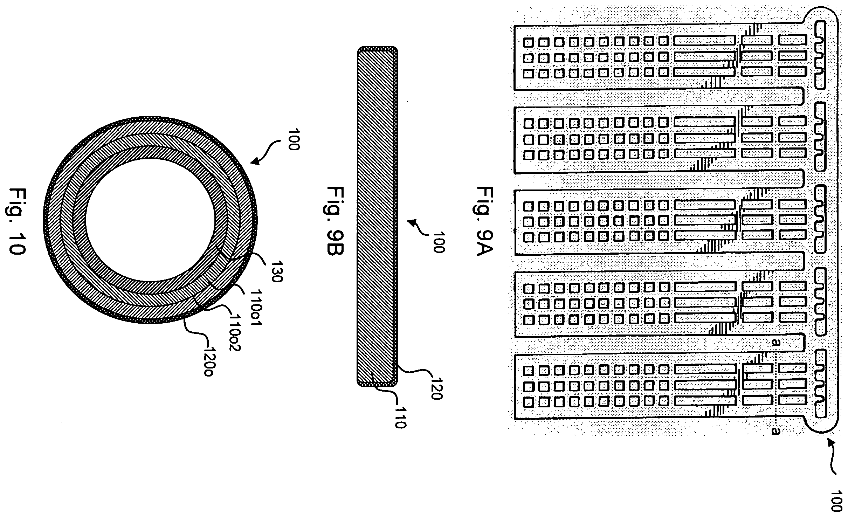

Device 100 illustrated in Fig. 10, which is not part of the invention, comprises a substrate 130, a first biodegradable polymeric region 110o1 on an outer surface of the substrate 130, a second biodegradable polymeric region 110o2 on an outer surface of the first biodegradable polymeric region 110o1, and a hard, thin surface layer 120o on an outer surface of the second biodegradable polymeric region 110o2. Such an embodiment may be advantageous, for instance, in drug delivery applications. For example, the first and second biodegradable polymeric regions 110o1, 110o2, may contain different drugs, such that the two drugs are delivered in a cascade, the regions 110o1, 110o2 may contain the same drug while being formed of different biodegradable materials, thereby achieving complex drug release profiles, and so forth.

One advantage of providing biodegradable polymeric regions with hard, thin surface layers in accordance with the invention is that such surfaces are amenable to promoting cell growth. Depending on the nature and location of the device, such cells may be, for instance, endothelial cells, muscle cells, connective tissue cells, and/or nerve cells, examples of which include among others: (a) squamous epithelial cells, such as non-keratinized squamous endothelial cells, for example, those lining the upper GI tract (e.g., cheek and esophagus) and lung alveoli, as well as the mesothelium lining of various major body cavities (e.g., peritoneal, pleural, pericardial) and the endothelium lining the heart, blood vessels, sinusoids and lymphatics, (b) cubodial epithelial cells, which frequently line glandular ducts, (c) columnar epithelial cells, such as those lining portions of the digestive tract (e.g., the stomach and small intestines), the female reproductive tract (e.g., the uterus and fallopian tubes), as well as numerous other body surfaces, (d) pseudostratified columnar epithelial cells, such as those lining portions of the respiratory tract (e.g., trachea) and ducts of the male reproductive system, (e) transitional epithelial cells, such as those lining the distensible walls of the urinary tract (e.g., the renal pelvis, ureters, bladder and urethra), (f) glandular epithelium, (g) smooth muscle cells, which lie beneath epithelial cells in many body lumens such as many of those found in the vasculature, the genitourinary system, respiratory tract, and gastrointestinal tract, (h) cardiomyocytes, and (i) connective tissue cells such as fibroblasts.

On the other hand, devices that are at least partially biodegradable are also advantageous in many instances. For example, the biodegradable portion(s) of the device may additionally provide the device with a desirable property (e.g., a mechanical or chemical property) upon administration to a subject, which property is not needed after a time (e.g., because its purpose has been served) and indeed may even become detrimental to the subject.

For example, the biodegradable portion(s) of the device may initially provide the device with mechanical strength and rigidity, which properties subsequently become unneeded at some point (e.g., as a result of the body's healing mechanisms) and may even become detrimental to the subject (e.g., because it impedes flexibility).

As another example, the biodegradable portion(s) of the device may initially provide the release of at least one therapeutic agent, which subsequently becomes unneeded at some point. For instance, it may be desirable to release at least one therapeutic agent from the device, up to and including essentially 100% of the therapeutic agent within the device (e.g., from 50% to 75% to 90% to 95% to 97% to 99% or more of the therapeutic agent) over the first 1 day to 2 days to 4 days to 1 week to 2 weeks to 5 weeks to 10 weeks to 25 weeks to 1 year or more of administration. One way to enhance release is to dissolve, disperse, or otherwise dispose the therapeutic agent within or beneath a biodegradable portion of the medical device.

As a specific example, upon implantation of vascular stents, it is desirable that such devices become covered with endothelial cells. Typically, cells prefer attachment to a hard surface. It is further desirable in some instances for the stent be biodegradable, for instance, because this property allows the blood vessel into which it is implanted to eventually return to (or at least approach) its native flexibility and/or because re-interventions can be performed without the burden of having a previously implanted stent structure in the way. These benefits of biodegradation are maximized with a fully biodegradable stent.

To the extent that surface endothelialization may be compromised by a fully biodegradable structure, biostable, hard, thin inner and outer layers are provided at the outside and inside surfaces of the tubular body of the medical device of the invention. After the bulk of the stent structure biodegrades, only a very thin residual hard, thin layer remains in such embodiments, thereby reducing the impact of the stent upon vessel flexibility and enhancing opportunities for re-intervention.

Advantages may also be realized in medical devices which have biostable substrates which are, however not part of the invention. For example, U.S. Patent App. Pub. No. 2003/0153971 to Chandrasekaran describes stent structures that comprise a metallic reinforcing component and a biodegradable polymeric material that covers at least a portion of the metallic reinforcing component and provides further mechanical reinforcement. Advantages of such structures include the following: (a) due to the presence of the metallic component, such stents are typically reduced in cross-section relative to stents that are composed entirely of biodegradable polymer, improving ease of implantability, (b) release of therapeutic agent, if present, is promoted, and (c) reduced amounts of metallic component remain after degradation of the biodegradable polymeric material covering, thereby increasing flexibility of the stent structure over time and reducing any metal-associated adverse properties. In accordance with an embodiment of the invention, such stent structures may be provided with a hard, thin layer on their inner surfaces, their outer surfaces, or both.

As used herein a "polymeric region" is region that contains one or more polymers, for example, 50 wt% or more, 75 wt% or more, 90 wt% or more, or even 95 wt% or more polymers.

As is well known, "polymers" are molecules containing multiple copies (e.g., 5 to 10 to 100 to 1000 to 10,000 or more copies) of one or more constitutional units, commonly referred to as monomers. Polymers may take on a number of configurations, which may be selected, for example, from linear, cyclic, branched and networked (e.g., crosslinked) configurations. Branched configurations include star-shaped configurations (e.g., configurations in which three or more chains emanate from a single branch point, such as a seed molecule), comb configurations (e.g., configurations having a main chain and a plurality of side chains), dendritic configurations (e.g., arborescent and hyperbranched polymers), and so forth. As used herein, "homopolymers" are polymers that contain multiple copies of a single constitutional unit. "Copolymers" are polymers that contain multiple copies of at least two dissimilar constitutional units, examples of which include random, statistical, gradient, periodic (e.g., alternating), and block copolymers.

Examples of biodegradable polymers for use in the present invention may be selected from suitable members of the following, among many others: (a) polyester homopolymers and copolymers such as polyglycolide, poly-L-lactide, poly-D-lactide, poly-D,L-lactide, poly(beta-hydroxybutyrate), poly-D-gluconate, poly-L-gluconate, poly-D,L-gluconate, poly(epsilon-caprolactone), poly(delta-valerolactone), poly(p-dioxanone), poly(trimethylene carbonate), poly(lactide-co-glycolide) (PLGA), poly(lactide-co-delta-valerolactone), poly(lactide-co-epsilon-caprolactone), poly(lactide-co-beta-malic acid), poly(lactide-co-trimethylene carbonate), poly(glycolide-co-trimethylene carbonate), poly(beta-hydroxybutyrate-co-beta-hydroxyvalerate), poly[1,3-bis(p-carboxyphenoxy)propane-co-sebacic acid], poly(sebacic acid-co-fumaric acid), and poly(ortho esters) such as those synthesized by copolymerization of various diketene acetals and diols, among others, (b) polyanhydride homopolymers and copolymers such as poly(adipic anhydride), poly(suberic anhydride), poly(sebacic anhydride), poly(dodecanedioic anhydride), poly(maleic anhydride), poly[1,3-bis(p-carboxyphenoxy)methane anhydride], and poly[alpha,omega-bis(p-carboxyphenoxy)alkane anhydrides] such as poly[1,3-bis(p-carboxyphenoxy)propane anhydride] and poly[1,3-bis(p-carboxyphenoxy)hexane anhydride], among others; and (c) amino-acid-based homopolymers and copolymers including tyrosine-based polyarylates (e.g., copolymers of a diphenol and a diacid linked by ester bonds, with diphenols selected, for instance, from ethyl, butyl, hexyl, octyl and bezyl esters of desaminotyrosyl-tyrosine and diacids selected, for instance, from succinic, glutaric, adipic, suberic and sebacic acid), tyrosine-based polycarbonates (e.g., copolymers formed by the condensation polymerization of phosgene and a diphenol selected, for instance, from ethyl, butyl, hexyl, octyl and bezyl esters of desaminotyrosyl-tyrosine), and leucine and lysine-based polyester-amides; specific examples of tyrosine based polymers include poly(desaminotyrosyl-tyrosine ethyl ester adipate) or poly(DTE adipate), poly(desaminotyrosyl-tyrosine hexyl ester succinate) or poly(DTH succinate), poly(desaminotyrosyl-tyrosine ethyl ester carbonate) or poly(DTE carbonate), poly(desaminotyrosyl-tyrosine butyl ester carbonate) or poly(DTB carbonate), poly(desaminotyrosyl-tyrosine hexyl ester carbonate) or poly(DTH carbonate), and poly(desaminotyrosyl-tyrosine octyl ester carbonate) or poly(DTO carbonate).

Examples of hard, thin materials include various metals, metal oxides, metal nitrides, metal carbides, metal carbonitrides, carbon, and combinations thereof. For example, the hard, thin material may comprise from less than 5 to 10 to 25 to 50 to 75 to 90 to 95 to 97.5 to 99 wt% of one, two, three, four, or more of these materials.

Specific examples of hard, thin materials include (a) biostable metals such as iron, and mixed metals (i.e., metal alloys) such as cobalt-chromium-aluminum-yttrium (CoCrAlY), nickel-aluminum (NiAl) and nickel-chromium-boron-silicon (NiCrBSi), among others, (b) biostable and including single and mixed metal oxides including iron oxide. aluminum oxide, zirconium oxide and titanium oxide, among others, (c) metal nitrides including single and mixed metal nitrides such as titanium nitride, chromium nitride, zirconium nitride, boron nitride, tantalum nitride, niobium nitride, silicon nitride, vanadium nitride, titanium aluminum nitride, titanium zirconium nitride and silicon titanium nitride, among others, (d) metal carbides including single and mixed metal carbides such as boron carbide, titanium carbide, chromium carbide, molybdenum carbide, niobium carbide, silicon carbide, tantalum carbide, tungsten carbide, vanadium carbide and zirconium carbide, among others, and (e) metal carbonitrides including single and mixed metal carbonitrides such as titanium carbonitride and zirconium carbonitride among others. Many of these and other materials are available from Williams Advanced Materials, NY, USA. Many of these materials can be deposited, for example, using processes such as those described below, including physical vapor deposition and ionic deposition, among other processes. Examples of hard, thin polymeric materials include mixtures of starch with poly(ethylene-vinyl-alcohol) or with poly(lactic acid), which have a modulus in the range of 200-800 MPa, and which are currently used for tissue engineering. See N.M. Neves, Materials Science and Engineering C, 25 (2005) 195-200.

Beneficial carbon materials include diamond-like carbon. As used herein, a "diamond-like carbon" material is one that contains a mixture of sp² (as in graphite) and sp³ (as in diamond) bonded carbon. Diamond-like carbon is generally hard, amorphous, and chemically inert. Diamond-like carbon is known to be biocompatible and is relatively non-conductive. Diamond-like carbon may contain, for example, from 50 mol% to 75 mol% to 90 mol% to 95 mol% to 97.5 mol% to 99 mol% or more carbon atoms. Hence, these layers may contain other elements besides carbon (e.g., dopants, impurities, etc.), including H, O, N, among many others.

Properties of diamond-like carbon typically vary with the ratio of sp³ to sp² bonding. For example, a variation in the sp³ fraction (i.e., the number of sp³ carbons ÷ (the number of sp³ carbons + the number of sp² carbons) from 10% to 80% has been reported to correspond to a change in hardness from about 10 GPa to about 90 GPa. Diamond-like carbon for use in the present invention may comprise an sp³ fraction ranging from 10% or less to 20% to 30% to 40% to 50% to 60% to 70% to 80% to 90% or more. In this regard, the term "tetrahedral amorphous carbon" (*ta-C)* is sometimes used to refer to diamond-like carbon with a high degree of sp³ bonding (e.g., 80% or more).

Diamond-like layers may be quite thin, ranging, for example, from 5 nm up to several µm, more typically ranging from 10 nm to 25 nm to 50 nm to 100 nm to 250 nm to 500 nm in thickness.

As noted above, in some embodiments, one or more therapeutic agents may be provided, for example, within or beneath the biodegradable polymeric regions of the medical devices of the present invention. "Therapeutic agents", "pharmaceuticals," "pharmaceutically active agents", "drugs" and other related terms may be used interchangeably herein and include genetic therapeutic agents, non-genetic therapeutic agents and cells. Therapeutic agents may be used singly or in combination.

Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan and telmisartan), and antispasmodic/anticholinergic drugs (e.g., oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Preferred non-genetic therapeutic agents include paclitaxel (including particulate forms thereof, for instance, protein-bound paclitaxel particles such as albumin-bound paclitaxel nanoparticles, e.g., ABRAXANE), sirolimus, everolimus, tacrolimus, zotarolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Exemplary genetic therapeutic agents for use in connection with the present invention include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP), SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonist such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PG12 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin (sirolimus) and its analogs (e.g., everolimus, tacrolimus, zotarolimus, etc.), cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925 assigned to NeoRx Corporation.

Numerous techniques are available for forming biodegradable polymeric regions in accordance with the present invention.

For example, where a polymeric region is formed from one or more polymers having thermoplastic characteristics, a variety of standard thermoplastic processing techniques may be used to form the polymeric region. Using these techniques, a polymeric region can be formed, for instance, by (a) first providing a melt that contains polymer(s) and any supplemental agents such as therapeutic agent(s), etc. and (b) subsequently cooling the melt. Examples of thermoplastic processing techniques, including compression molding, injection molding, blow molding, spraying, vacuum forming and calendaring, extrusion into sheets, fibers, rods, tubes and other cross-sectional profiles of various lengths, and combinations of these processes. Using these and other thermoplastic processing techniques, entire devices or portions thereof can be made.

Other processing techniques besides thermoplastic processing techniques may also be used to form the polymeric regions of the present invention, including solvent-based techniques. Using these techniques, a polymeric region can be formed, for instance, by (a) first providing a solution or dispersion that contains polymer(s) and any supplemental agents such as therapeutic agent(s), etc., and (b) subsequently removing the solvent. The solvent that is ultimately selected will contain one or more solvent species, which are generally selected based on their ability to dissolve at least one of the polymer(s) that form the polymeric region, in addition to other factors, including drying rate, surface tension, etc. In certain instances, the solvent is selected based on its ability to dissolve and supplemental agents, if any, as well. Solvent-based techniques include, but are not limited to, solvent casting techniques, spin coating techniques, web coating techniques, solvent spraying techniques, dipping techniques, techniques involving coating via mechanical suspension including air suspension, ink jet techniques, electrostatic techniques, and combinations of these processes.

In a specific example, an entire medical device is extruded.

In accordance with the present invention, hard, thin layers for use in the present invention may be formed at surfaces of biodegradable polymeric regions using a variety of deposition and/or implantation techniques, including thermoplastic and solvent processing techniques (e.g., where the hard, thin layer is a polymeric layer), physical vapor deposition, ion deposition, ion implantation, chemical vapor deposition, and combinations thereof. These processes are typically conducted in the presence of a substrate, in this case, one comprising a biodegradable polymeric region.

Physical vapor deposition, ion deposition and ion implantation are typically conducted under vacuum (i.e., at pressures that are less than ambient atmospheric pressure). By providing a vacuum environment, the mean free path between collisions of vapor particles (including atoms, molecules, ions, etc.) is increased and the concentration of gaseous contaminants is reduced, among other effects.

Physical vapor deposition (PVD) processes are processes in which a source of material, typically a solid material, is vaporized and transported to a substrate where a film (i.e., a layer) of the material is formed. PVD can take place in a wide range of gas pressures, for example, commonly within the range of 10⁻⁵ to 10⁻⁹ Torr, among other pressure ranges. In many embodiments, the pressure associated with PVD techniques is sufficiently low such that little or no collisions occur between the vaporized source material and ambient gas molecules while traveling to the substrate. Hence, the trajectory of the vapor is a substantially straight (line-of-sight) trajectory. Many PVD processes are low temperature (including room temperature) processes, which is desirable when dealing with thermally sensitive materials such as various biodegradable polymers and, in some embodiments, various therapeutic agents.

Some specific PVD methods that may be used to form hard, thin layers in accordance with the present invention include evaporation, sublimation, sputter deposition and laser ablation deposition. For instance, in some embodiments, at least one source material is evaporated or sublimed, and the resultant vapor travels from the source to a substrate, resulting in a deposited layer on the substrate. Examples of sources for these processes include resistively heated sources, heated boats and heated crucibles, among others. Sputter deposition is another PVD process, in which surface atoms or molecules are physically ejected from a surface by bombarding the surface (commonly known as a sputter target) with high-energy ions. As above, the resultant vapor travels from the source to the substrate where it is deposited. Ions for sputtering can be produced using a variety of techniques, including arc formation (e.g., diode sputtering), transverse magnetic fields (e.g., magnetron sputtering), and extraction from glow discharges (e.g., ion beam sputtering), among others. One commonly used sputter source is the planar magnetron, in which a plasma is magnetically confined close to the target surface and ions are accelerated from the plasma to the target surface. Laser ablation deposition is yet another PVD process. It is similar to sputter deposition, except that vaporized material is produced by directing laser radiation (e.g., pulsed laser radiation), rather than high-energy ions, onto a source material (typically referred to as a target). The vaporized source material is subsequently deposited on the substrate.

In accordance some embodiments of the invention, two or more materials are codeposited using any of several PVD processes, including evaporation, sublimation, laser ablation and sputtering. For instance, two or more materials can be co-sputtered (e.g., by sputtering separate targets of each of the materials or by sputtering a single target containing multiple materials, for example, a metal alloy target), among many other possibilities.

Materials available for physical vapor deposition include single metals and mixed metal materials (i.e., metal alloys), single and mixed metal oxides, single and mixed metal nitrides, single and mixed metal carbides, single and mixed metal carbonitrides, and polymers (e.g., using pulsed laser deposition), for example, selected from those listed above, among others.

Hard, thin layers may also be formed by ion deposition processes. An "ion deposition process" is a deposition process in which ions are accelerated by an electric field, such that the substrate is bombarded with ions during the deposition process.

In some instances, the substrate is bombarded with ions during the course of a PVD deposition process, in which case the technique is sometimes referred to as ion beam assisted deposition. For example, the substrate can be bombarded with ions of a reactive gas such as oxygen or nitrogen, or an inert gas such as argon, during the course of a PVD process like those discussed above. These ions can be provided, for example, by means of an ion gun or another ion beam source.

In some instances, at least a portion of the deposition vapor itself is ionized and accelerated to the substrate. For example, the deposition vapor can correspond to the material to be deposited (e.g., where a vapor produced by a PVD processes such as evaporation, sublimation, sputtering or laser ablation is ionized and accelerated to the substrate). Deposition vapors can be ionized using a number of techniques. For example, deposition vapor can be at least partially ionized by passing the same through a plasma. Plasmas may be produced, for example, by DC hot cathode (filaments) or magnetron discharges, by RF discharges (e.g., sustained at 13.56 MHz), or by ECR (electron cyclotron resonance) discharges (e.g., sustained at 2.45 GHz), among other processes. As another example, partially ionized vapor can be directly generated at a material source, for instance, by subjecting the material source to an arc erosion process, such as cathodic or anodic arc erosion processes.

Other aspects of the present invention are directed to the formation of hard, thin layers on biodegradable polymer surfaces using methods that comprise CVD. CVD is a process whereby atoms or molecules are deposited in association with a chemical reaction (e.g., a reduction reaction, an oxidation reaction, a decomposition reaction, etc.) of vapor-phase precursor species. When the pressure is less than atmospheric pressure, the CVD process is sometimes referred to as low-pressure CVD or LPCVD. Plasma-enhanced chemical vapor deposition (PECVD) techniques are chemical vapor deposition techniques in which a plasma is employed such that the precursor gas is at least partially ionized, thereby reducing the temperature that is required for chemical reaction. In some embodiments, the ionized vapor phase precursor species are accelerated to the substrate.

In certain embodiments of the invention, an ionic species is subjected to an electric field that is sufficiently large such that the ions impacting the biodegradable polymer convert the surface region of the biodegradable polymer into a hard, thin layer. Such processes are commonly referred to as "ion implantation" processes. Suitable species for ion implantation include, for example, reactive and non-reactive species (e.g., a reactive gas such as oxygen or an inert gas such as argon, heliuim, nitrogen, etc.).

As indicated above, in certain embodiments of the invention, the hard, thin layer is a carbonaceous layer such as a diamond-like carbon layer. Techniques for forming carbonaceous layers include deposition techniques, implantation techniques, or combinations of both. These processes may involve, for example, deposition and/or implantation of energized ions (e.g., 10-500 eV). Because the layer formed shares an interface (which may involve a gradual or abrupt transition) with a biodegradable polymeric region, preferred techniques are those which do not subject the bulk of the polymeric region to excessively high temperatures.

Several reported examples of techniques that have been used to form carbonaceous films, including diamond-like carbon (DLC) films, follow. These include deposition-based techniques such as sputter deposition, gas cluster ion beam assisted deposition, filtered cathodic arc deposition, and plasma-enhanced chemical vapor deposition, among others.

For example, D. W. Han et al. report the formation of a DLC film on poly(2-methoxy-5-(2'-ethylhexoxy)-1,4-phenylenevinylene) (MEH-PPV) using a Cs⁺ ion gun sputter deposition system. Negative carbon ion energy varied from 50 to 200 eV, and the sp²/sp³ ratio was controlled by changing the carbon ion energy. See D. W. Han et al., "Electron injection enhancement by diamond-like carbon film in organic electroluminescence devices," Thin Solid Films, 420-421 (2002) 190-194, and the references cited therein.

U.S. Pat. No. 6,416,820 to Yamada et al. describes a method for forming a carbonaceous hard film that includes vapor depositing a hard film of a carbonaceous material onto a substrate by vacuum deposition of a vaporized, hydrogen-free carbonaceous material, which may be ionized or non-ionized, onto the substrate surface, while irradiating the carbonaceous material with gas cluster ions, generated by ionizing gas clusters to form the film. Yamada et al. report that there is no need to heat the substrate.

T. Kitagawa et al., "Study of Ar Cluster Ion Incident Angle for Super Hard Diamond Like Carbon Film Deposition," UVSOR Activity report 2003, B1BL8, describe the deposition of super-hard (> 50 GPa) DLC thin films with a smooth surfaces and low sp²orbital content at room temperature by Ar gas cluster ion beam (GCIB) assisted deposition using fullerene as the carbon source. See also K Kanda et al. "Characterization of Hard Diamond-Like Carbon Films Formed by Ar Gas Cluster Ion Beam-Assisted Fullerene Deposition," Jpn. J. Appl. Phys. Vol. 41 (2002) 4295-4298, T. Kitagawa et al., "Optimum Incident Angle of Ar Cluster Ion Beam for Superhard Carbon Film Deposition," Jpn. J. Appl. Phys. Vol. 43, No. 6B, 2004, pp. 3955-3958 and T. Kitigawa et al., "Near Edge X-Ray Absorption Fine Structure Study for Optimization of Hard Diamond-Like Carbon Film Formation with Ar Cluster Ion Beam," Jpn. J. Appl. Phys. Vol. 42 (2003) 3971-3975 Part 1, No. 6B, 30 June 2003.

E. Amanatides et al., "Electrical and optical properties of CH4/H2 RF plasmas for diamond-like thin film deposition," Diamond & Related Materials 14 (2005) 292- 295, describe the deposition of DLC on PVC foils from CH₄/H₂ using plasma-enhanced chemical vapor deposition (PE-CVD). The authors note that PE-CVD is advantageous because it permits the deposition on polymer substrates, even at room temperature. See also W.S. Choi et al., "Synthesis and characterization of diamond-like carbon protective AR coating," Journal of the Korean Physical Society, Vol. 45, December 2004, pp. S864-S867 in which DLC films were deposited at room temperature by PE-CVD.

M. Tonosaki et al., in "Nano-indentation testing for plasma-based ion-implanted surface of plastics," Surf. Coat. Technol., vol. 136, pp. 249-251, 2001, used a filtered cathodic arc as a carbon ion source and supplied bipolar pulses to improve the hardness of amorphous polyolefin. A surface Young's modulus of 25 GPa was reported. In filtered cathodic arc deposition a solid target is evaporated by an arc discharge. A magnetic field is applied to carry ionized particles around a bend, and the ion energy at the substrate can be controlled by applying a bias voltage. Ion bombardment has been shown to improve the quality of films produced by filtered cathodic arc deposition. See M. L. Fulton, "Ion-Assisted Filtered Cathodic Arc Deposition (IFCAD) System for Volume Production of Thin-Film Coatings," Society of Vacuum Coaters, 42nd Annual Technical Conference Proceedings (1999).

Another example of a deposition-implantation technique is plasma immersion ion implantation-deposition (PIII-D). For instance, J.Y. Chen et al., "Blood compatibility and sp3/sp2 contents of diamond-like carbon (DLC) synthesized by plasma immersion ion implantation-deposition," Surface and Coatings Technology 156 (2002) 289-294 describe the use of plasma immersion ion implantation-deposition (PIII-D) in the fabrication of DLC films on silicon substrates at room temperature. The sp³/sp² ratio (and platelet adhesion) of the film was varied by changing the C₂H₂ to Ar flow ratio during deposition. See also X-M He et al., Journal of Vacuum Science & Technology B: Microelectronics and Nanometer Structures, Volume 17, Issue 2 (March 1999) pp. 822-827, in which DLC films were prepared on low temperature substrates such as poly(methylmethacrylate) (PMMA) using the C₂H₂-Ar plasma immersion ion processing.

Other techniques rely solely on ion implantation to convert the surface region of the biodegradable polymer into a hard, thin layer. An example of such a technique is plasma immersion ion implantation (PIII). In such techniques, ions generated in a plasma are bombarded onto a substrate.

Where insulators are being treated, problems can be encountered as a result of a potential drop across the sample, which may be so severe that no implantation occurs. This problem has been explained in terms of capacitance and surface charging effects, which lead, for example, to electrical arcing and decreased ion energy. To address this problem, so-called "mesh assisted" techniques have been employed in which a conductive grid is placed over the sample and in electrical contact with an underlying conductive substrate holder. Consequently, ions are accelerated toward the grid and pass through the holes where they are implanted into the insulator surface. The size of the grid holes is adjusted to optimize ion energy and dose uniformity. See e.g., P.K. Chu, "Recent developments and applications of plasma immersion ion implantation," J Vac. Sci. Technol. B 22(1), Jan/Feb 2004, 289-296. Such grids are known to create shadow effects, which can be addressed by moving the sample relative to the grid (e.g., either during implantation or between implantation steps). In some embodiments, however, grid effects are desirable to the extent that they form apertures in the carbonaceous layer which can promote degradation of the underlying biodegradable region as discussed above.

Further information on mesh-assisted PIII can be found, for example, in P.K. Chu, "Recent developments and applications of plasma immersion ion implantation," J Vac. Sci. Technol. B 22(1), Jan/Feb 2004, 289-296, R.K.Y. Fu et al., "Effects of mesh-assisted carbon plasma immersion ion implantation on the surface properties of insulating silicon carbide ceramics," J. vac. Sci. Technol. A 22(2), Mar/Apr 2004, 356-360; R.K.Y. Fu et al., "Influence of thickness and dielectric properties on implantation efficacy in plasma immersion ion implantation of insulators," J. Appl. Phys., Vol. 95, No. 7, 1 April 2004, 3319-3323.

Applied voltages during PIII of biodegradable polymeric regions may range, for example, from 10kV to 100kV, with pulse duration ranging from 1-100 µs at a frequency ranging from 10 to 1000 Hz. Bombarding species include, for example, inert species such as argon, helium and nitrogen ions, among others. In general, the ratio of sp³ hybridized carbon to sp²hybridized carbon increases with increasing dose. Typical dosages may range, for example, from 10¹⁵ to 10¹⁷ ions per cm² , among other possibilities. An increase in energy will generally result in an increase in thickness of the carbonaceous layer that is formed. Typical energies may range, for example, from 10 keV to 50 keV, among other possibilities.

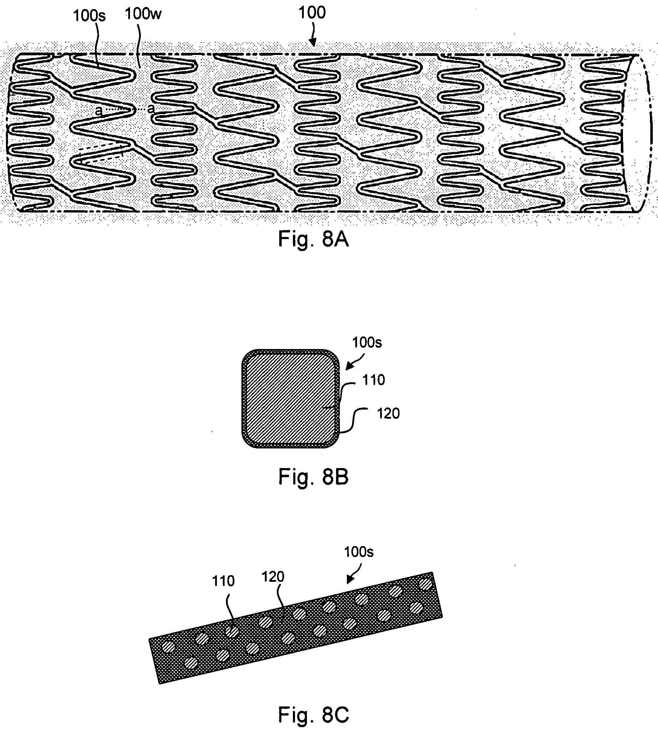

Fig. 8A illustrates a stent body 100, analogous in design to that described in U.S. Patent Pub. No. 2004/0181276, and comprises various struts 100s. Unlike the stent of U.S. Patent Pub. No. 2004/0181276, however, stent body 100 is constructed to in accordance with the present invention. For example, the stent body 110 may have a hard, thin layer on its outer and inner surfaces.

For example, Fig. 8B is a schematic cross-sectional view of a stent strut 100s taken along line a--a of Fig. 8A. As seen from Fig. 8B the stent strut 100s comprises an inner biodegradable region 110 and an outer hard, thin region 120. Fig. 8C is an expanded top view of the rectangular region defined by dashed lines in Fig. 8A. As seen from Fig. 8C the outer hard, thin region 120 on the stent strut 110s is provided with apertures through which the inner biodegradable region 110 is exposed to the environment (e.g., bodily fluid) surrounding the stent.

Where line-of-sight deposition and/or implantation techniques are employed to create the hard, thin layer 120, both the inner and outer surfaces of the stent may be covered with the hard, thin layer 120, for instance, by moving (e.g., rotating, tilting, etc.) the stent 100 in a continuous or stepwise fashion during processing. The hard, thin layer 120 is formed on the inner surface of the stent 100, because species for deposition/implantation are above to pass from the exterior to the interior of the device through the open spaces 100w that are present between the struts 100s. Apertures may be formed in the hard, thin layer 120, for example, using techniques described below.

In the event that it is desired to form a hard, thin layer on only the outer surface of the stent, an embodiment which is not part of the invention, the stent may be mounted on a mandrel or another support which acts to prevent species from passing through the open spaces 100w and striking the interior surface of the device. A hard, thin layer may be formed only on the inner surface of the stent, an embodiment which is not part of the invention, by masking the inner surface of the stent after depositing a hard, thin layer over the entire device, followed by etching of the outer layer and mask removal. As another example, in a process called interior plasma vapor deposition, a PVD source may be situated in the center of a cylindrical stent so as to only coat the inner surface of the same. Of course, for such a process to succeed, the source must be sufficiently small, relative to the size of the stent.

A stent with a hard, thin layer on both its inner and outer surfaces may also be created by first forming a hard, thin layer on both surfaces of a planar sheet of biodegradable polymer. This planar sheet is then subsequently rolled to form a tubular member corresponding to a stent or a portion thereof. Stents of this nature are described, for example, in U.S. Pat. Pub. No. 2001/0044651 to Steinke et al. and U.S. Patent No. 5,649,977 to Campbell. Fig. 9A illustrates a planar sheet 100, which is analogous in design to that described in U.S. Patent No. 5,649,977 to Campbell. Unlike the planar sheet of U.S. Patent No. 5,649,977 to Campbell, however, the sheet 100 illustrated is constructed in accordance with the present invention. For example, the planar sheet 110 may have a hard, thin layer on its and its upper and lower surfaces. As an example, in the schematic cross-sectional view of Fig. 9B, which is taken along line a--a of Fig. 9A, a planar sheet 100 is shown which comprises a biodegradable bulk region 110 and a hard, thin region 120 on its upper surface. The hard, thin region 120 may be on either the inner or outer surface of the stent that is formed from the planar sheet 100, depending upon which way the planar sheet 100 is This embodiment is, however not part of the present invention.

As noted above, apertures are provided in the hard, thin inner layer. For example, apertures may be creating by forming a hard, thin material layer over only certain portions of an underlying biodegradable polymer region or by removing certain portions of a hard, thin material once formed.

For instance, hard, thin material may be selectively formed in certain regions by directing a focused beam of material (e.g., a focused beam of ions) onto the biodegradable material (e.g., for purposes of deposition and/or implantation). Apertures may also be formed by masking a portion of the biodegradable material such that the hard, thin layer is not formed in certain areas. Mask-based techniques include those in which the masking material contacts the biodegradable material, for example, masks formed using known lithographic techniques, including optical, ultraviolet, deep ultraviolet, electron beam, and x-ray lithography, and those in which the masking material does not contact the biodegradable material, but is instead provided between a source of layer-creating material (e.g., species for deposition and/or implantation) and the biodegradable material.

Examples of techniques by which hard, thin materials may be selectively removed (i.e., machined) include direct-write techniques, as well as mask-based techniques in which masking is used to protect portions of the machined layers that are not excavated.

Direct write techniques include those in which excavated regions are created through contact with solid tools (e.g., microdrilling, micromachining, etc., using high precision equipment such as high precision milling machines and lathes) and those that form excavated regions without the need for solid tools (e.g., those based on directed energetic beams, for example, laser ablation). In the latter cases, techniques based on diffractive optical elements (DOEs), holographic diffraction, and/or polarization trepanning, among other beam manipulation methods, may be employed to generate direct-write patterns as desired. Using these and other techniques, many apertures can be ablated in a material layer at once. Further information on laser ablation may be found in Lippert T, and Dickinson JT, "Chemical and spectroscopic aspects of polymer ablation: Special features and novel directions," Chem. Rev., 103(2): 453-485 Feb. 2003; Meijer J, et al., "Laser Machining by short and ultrashort pulses, state of the art and new opportunities in the age of photons," Annals of the CIRP, 51 (2), 531-550, 2002, and U.S. Patent No. 6,517,888 to Weber.

Where laser radiation is used to form apertures in the hard, thin layer, manufacturing tolerances typically are on the order of the wavelength of the laser. However, as recently shown in K. Konig et al., Medical Laser Application 20 (2005) 169-184, materials may be ablated on the order of 1/15th of the optical wavelength (as demonstrated with a 800 nm ultrashort pulse laser), allowing the formation of holes and trenches in the nanometer range. Consequently, laser radiation can be directed into very small areas, allowing, for example, one to create apertures within small device components, for example, stent struts, among many other possibilities. For example, apertures of 1 µm² or less in area may be formed, which apertures are much smaller than many cells.

Mask-based techniques, like those described above for use in selectively forming hard, thin regions, include those in which the masking material contacts the layer to be machined, for example, masks formed using known lithographic techniques, and those in which the masking material does not contact the layer to be machined, but which is provided between a directed source of excavating energy and the material to be machined (e.g., opaque masks having apertures formed therein, as well as semi-transparent masks such as gray-scale masks which provide variable beam intensity and thus variable machining rates). Material is removed in regions not protected such by such masks using any of a range of processes including physical processes (e.g., thermal sublimation and/or vaporization of the material that is removed), chemical processes (e.g., chemical breakdown and/or reaction of the material that is removed), or a combination of both. Specific examples of removal processes include wet and dry (plasma) etching techniques, and ablation techniques based on directed energetic beams.

## Claims

1. An implantable or insertable medical device (100) comprising:
a biodegradable polymeric region (110) comprising a tubular body,
an inner hard, thin biostable layer (120i) disposed over the entire surface of said biodegradable polymeric region (110) by covering the inside surface of said tubular body, wherein said inner hard, thin biostable layer is provided with a plurality of apertures (120a), and
an outer hard, thin biostable layer (120o) covering the outside surface of said tubular body.

2. The implantable or insertable medical device of claim 1, wherein said medical device (100) is a vascular medical device.

3. The implantable or insertable medical device of claim 1, wherein said medical device (100) is selected from a stent.

## Patentansprüche

1. Implantierbare oder einführbare medizinische Vorrichtung (100), umfassend:
eine biologisch abbaubare Polymerregion (110), die einen röhrenförmigen Körper umfasst,
eine innere harte, dünne biostabile Schicht (120i), die über der gesamten Oberfläche der biologisch abbaubaren Polymerregion (110) angeordnet ist, indem sie die Innenfläche des röhrenförmigen Körpers bedeckt, wobei die innere harte, dünne biostabile Schicht mit einer Vielzahl von Öffnungen (120a) versehen ist, und
eine äußere harte, dünne biostabile Schicht (120o), die die Außenfläche des röhrenförmigen Körpers bedeckt.

2. Implantierbare oder einführbare medizinische Vorrichtung nach Anspruch 1, wobei die medizinische Vorrichtung (100) eine vaskuläre medizinische Vorrichtung ist.

3. Implantierbare oder einführbare medizinische Vorrichtung nach Anspruch 1, wobei die medizinische Vorrichtung (100) unter einem Stent ausgewählt ist.

## Revendications

1. Dispositif (100) médical implantable ou insérable comprenant :
une zone (110) polymère biodégradable comprenant un corps tubulaire,
une couche (120i) interne dure et fine biostable disposée sur la surface entière de ladite zone (110) polymère biodégradable par recouvrement de la surface intérieure dudit corps tubulaire, dans lequel ladite couche interne dure et fine biostable est pourvue d'une pluralité d'ouvertures (120a), et
une couche (120o) externe dure et fine biostable recouvrant la surface extérieure dudit corps tubulaire.

2. Dispositif médical implantable ou insérable selon la revendication 1, dans lequel ledit dispositif (100) médical est un dispositif médical vasculaire.

3. Dispositif médical implantable ou insérable selon la revendication 1, dans lequel ledit dispositif (100) médical est choisi parmi un tuteur.
